# EUROPEAN PATENT APPLICATION

(11) **EP 1 992 287 A1**
(43) Date of publication of application: **19.11.2008**
(21) Application number: 07737887.5
(22) Date of filing: 06.03.2007
(51) Int. Cl.: A61B 6/03, A61B 5/055

(54) **MEDICAL IMAGE DISPLAY METHOD AND PROGRAM THEREOF**

(30) Priority: 09.03.2006 JP 2006064623
(71) Applicant: Imagnosis Inc., Kobe-shi, Hyogo 658-0032 (JP)
(72) Inventor: KIM, Han-Joon, Kobe-shi, Hyogo, 6580032 (JP)
(74) Representative: Steil, Christian
(86) International application number: PCT/JP2007/054340
(87) International publication number: WO 2007/105545

(57) **Abstract**

It has been difficult to display medical three-dimensional images taken by different imaging devices (imaging methods or modalities) individually in a common display direction. In the invention, a coordinate system 1 on the basis of landmarks on the bones and a coordinate system 2 on the basis of landmarks on the skins are set in a CT image, and a transformation coefficient (correction amount) Tc between the coordinate system 1 and the coordinate system 2 is calculated and stored. In a case where image data acquired by another imaging method is displayed, a coordinate system same as the coordinate system 1 in the CT image can be set in a case where the image data includes a bone display layer, and a coordinate system same as the coordinate system 2 in the CT image can be set in a case where the image displays a skin display layer. By correcting the coordinate system thus set using the transformation coefficient Tc, the coordinate system 1 can be corrected to the coordinate system 2 or the coordinate system 2 to the coordinate system 1. It is thus possible to display images according to the common coordinate system between different sets of image data.

## Description

### Technical Field

The present invention relates to the display of medical images, and more particularly, to a method and a program for displaying medical images by setting a common coordinate system that determines a display direction of images and displaying medical images according to this coordinate system.
Three-dimensional medical image data acquired by CT, MRI, and so forth has been attracting attention for use in three-dimensional image diagnoses in the medical field, and is now actively applied to clinical care.
Regarding the medical three-dimensional image data, however, when the imaging devices or the imaging methods are different, so are the coordinate systems of the acquired image data (in other words, the display directions and extracted contents of the image data). For example, a CT image can show bones alone or skins alone three-dimensionally. Accordingly, in order to correct the display direction of images, a common coordinate system can be constructed using a plurality of landmarks (anatomical features) on the bones or on the skins.

On the contrary, the landmarks on the bones cannot be extracted on an MRI image. In other words, an MRI image shows the soft tissues, such as skins, but it does not show the hard tissues, such as bones.
In addition, in SPECT, PET, and the like, because accumulated images of a tracer are the main, anatomical extraction (spatial resolution) is so poor that it is difficult to identify the landmarks.
Such being the case, it is difficult to display medical images-taken by different imaging devices or different imaging methods in a common display direction.

For example, because anatomical morphology extraction of a PET image is poor, even when an abnormality is found, it is difficult to understand the anatomical positional relation, "where the focus is present", using a PET image alone. To overcome this problem, when a PET image is evaluated, it is necessary to make a diagnosis by comparing the PET image with a CT or MRI image having good spatial resolution (see, for example, Non-Patent Document 1). In such a case, however, the display directions of the PET image and the CT or MRI image have to coincide with each other. To this end, an imaging device of a type capable of taking a PET image and a CT image simultaneously has been developed. However, this type has problems that the equipment is expensive and an exposure dose is increased.
Non-Patent Document 1: http://www.shiga-med.ac.jp/hospital/ houshasenbu/riweb/spect.htm
Patent Document 1: US Patent No.6,888,546

### Disclosure of the Invention

### Problems that the Invention is to Solve

It is general to perform a work to overlay PET data and CT data acquired separately to find the positional relation. However, the overlaying work has to be performed every single time. In addition, two sets of data are acquired separately and the extraction state of the soft tissues varies to some extent with the posture at the time of imaging. Such a variation possibly makes it difficult to overlay images along the entire outer shape or on the cross sections or extends a processing time needed for the overlaying and accompanying works. To overcome these problems, there has been adopted a method of constructing a coordinate system on the basis of immovable landmarks or contrast markers common between respective sets of data. However, extraction of anatomical landmarks remains poor in PET in any case, and it is still necessary to overlay a PET image and a CT image in order to form a PET image in which the anatomical direction is specified.

In short, because extracted images are different among the imaging methods, such as CT, MRI, SPECT, and PET, there has been a problem that it is difficult to display medical three-dimensional images taken by different imaging devices (imaging methods or modalities) individually in the common display direction.
The invention was devised to solve the problems discussed above, and has an object to provide a method and a display program for a computer for displaying medical images in the same direction at the same angle by setting a common display coordinate system for medical image data acquired by different imaging devices or imaging methods.

### Means for Solving the Problems

An invention set forth in claim 1 is a display method of a medical image according to image data acquired by different imaging methods, comprising the steps of: displaying a first image according to first medical three-dimensional image data of a patient acquired by a first imaging method; setting a first reference coordinate system used to determine a direction of the first image on the basis of a plurality of first landmarks included in the first image; setting a second reference coordinate system used to determine the direction of the first image on the basis of a plurality of second landmarks included in the first image and having at least one landmark different from the first landmarks; computing a difference of directions between the first reference coordinate system and the second reference coordinate system and calculating a transformation coefficient between the both coordinate systems on the basis of the difference; displaying a second image according to second medical three-dimensional image data of the patient acquired by a second imaging method; setting a third reference coordinate system used to determine a direction of the second image on the basis of a plurality of third landmarks included in the second image; correcting the third reference coordinate system to a fourth reference coordinate system using the transformation coefficient that has been calculated; and displaying the second image in a direction according to the fourth reference coordinate system set by correction.

An invention set forth in claim 2 is a display method of a medial image, comprising the steps of: preparing a transformation coefficient on the basis of a difference between a pre-calculated coordinate system in reference to landmarks on hard tissues represented by bones and a pre-calculated coordinate system in reference to landmarks on soft tissues represented by skins; constructing a first reference coordinate system used to determine a direction of an image on the basis of a plurality of landmarks on an image showing the soft tissues in a case where a medical image of a patient taken by a predetermined imaging method is an image that shows the soft tissues represented by the skins but does not show the hard tissues represented by the bones; correcting the first reference coordinate system to a second reference coordinate system using the transformation coefficient; and displaying an image of the patient in a direction according to the second reference coordinate system.

An invention set forth in claim 3 is a display method of a medical image according to image data acquired by different imaging methods, comprising the steps of: computing a difference between two coordinate systems set on first medical three-dimensional image data acquired by a first imaging method and calculating a transformation coefficient between the both coordinate systems on the basis of the difference; setting a coordinate system common with either one of the two coordinate systems on second medical three-dimensional image data acquired by a second imaging method; and correcting the coordinate system set on the second medical three-dimensional image data using the transformation coefficient that has been calculated.

An invention set forth in claim 4 is the display method of a medical image according to any one of claims 1 through 3, wherein the step of setting or constructing the coordinate system using a display image of anatomical landmarks or imaging markers displayed on the image.
An invention set forth in claim 5 is a medical image display program that causes a computer to display a medical image, comprising the steps of: displaying a first image according to first medical three-dimensional image data of a patient acquired by a first imaging method; setting a first reference coordinate system used to determine a direction of the first image on the basis of a plurality of first landmarks included in the first image; setting a second reference coordinate system used to determine the direction of the first image on the basis of a plurality of second landmarks included in the first image and having at least one landmark different from the first landmarks; computing a difference of directions between the first reference coordinate system and the second reference coordinate system and calculating a transformation coefficient between the both coordinate systems on the basis of the difference; displaying a second image according to second medical three-dimensional image data of the patient acquired by a second imaging method; setting a third reference coordinate system used to determine a direction of the second image on the basis of a plurality of third landmarks included in the second image; correcting the third reference coordinate system to a fourth reference coordinate system using the transformation coefficient that has been calculated; and displaying the second image in a direction according to the fourth reference coordinate system set by correction.

An invention set forth in claim 6 is a medical image display program that causes a computer to display a medical image, comprising the steps of: preparing a transformation coefficient on the basis of a difference between a pre-calculated coordinate system in reference to landmarks on hard tissues represented by bones and a pre-calculated coordinate system in reference to landmarks on soft tissues represented by skins; constructing a first reference coordinate system used to determine a direction of an image on the basis of a plurality of landmarks on an image showing the soft tissues in a case where a medical image of a patient taken by a predetermined imaging method is an image that shows the soft tissues represented by the skins but does not show the hard tissues represented by the bones; correcting the first reference coordinate system to a second reference coordinate system using the transformation coefficient; and displaying an image of the patient in a direction according to the second reference coordinate system.

An invention set forth in claim 7 is a medical image display program that causes a computer to display a medical image, comprising the steps of: computing a difference between two coordinate systems set on first medical three-dimensional image data acquired by a first imaging method and calculating a transformation coefficient between the both coordinate systems on the basis of the difference; setting a coordinate system common with either one of the two coordinate systems on second medical three-dimensional image data acquired by a second imaging method; and correcting the coordinate system set on the second medical three-dimensional image data using the transformation coefficient that has been calculated.

An invention set forth in claim 8 is the medical image display program that causes a computer to display a medical image according to any one of claims 5 through 7, wherein the step of setting or constructing the coordinate system using a display image of anatomical landmarks or imaging markers displayed on the image.

### Advantages of the Invention

According to the method or the program for displaying a medical image of the invention, the first image (CT image) is displayed according to the first medical three-dimensional data (CT data) of the patient acquired by the first imaging method (for example, CT).
The volume is then adjusted in the CT image being displayed so that, for example, a bone display layer is shown thereon. The first reference coordinate system (the reference coordinate system on the basis of the landmarks on the bones) that determines the direction of the CT image is then set by specifying more than one, for example, three landmarks on the bones. The CT image can be thus displayed in the display direction CT1 according to the first reference coordinate system.

Subsequently, the volume of the CT image is adjusted to display a skin display layer. The second reference coordinate system (the reference coordinate system on the basis of the landmarks on the skins) that determines the direction of the CT image is then set by specifying a plurality of landmarks on the skins. The CT image can be thus displayed in the display direction CT2 according to the second reference coordinate system.
Further, a difference between the first reference coordinate system and the second reference coordinate system is found by computation and the transformation coefficient Tc between the both coordinate systems is calculated.

Subsequently, when the second medical three-dimensional image data (MRI data) of the same patient is acquired by the second imaging method, for example, MRI, an MRI image is displayed.
The third reference coordinate system (this third reference coordinate system is in a direction corresponding to the display direction CT2 in the CT image) that determines the direction of the MRI image is then defined by specifying a plurality of landmarks on the skins displayed on the MRI image. The MRI image according to the third reference coordinate system is in the display direction MRI2.

Because bones are not shown on the MRI image, the reference coordinate system cannot be set by specifying landmarks on the bones. However, the third reference coordinate system is corrected using the transformation coefficient Tc that has been found in advance with respect to the third reference coordinate system, which is the display reference of the display direction MRI2. It is thus possible to construct the fourth reference coordinate system corresponding to the first reference coordinate system indicating the display direction CT1.

By displaying the MRI image according to the fourth reference coordinate system, it is possible to display the MRI image in the display direction MRI1 corresponding to the display direction CT1.
In other words, according to the method or the program of the invention, in medical image data acquired by different imaging devices or imaging methods, the coordinate system set on the basis of extracted landmarks or markers is defined as the reference coordinate system. This reference coordinate system is correlated with the respective coordinate systems in plural display directions on the imaging data, such as CT data and MRI data, whose anatomical directions can be set in various manners. Accordingly, as long as the data is acquired from the same patient, even when data is acquired by a different imaging device or imaging method, it is possible to display an image in a specific display direction by correcting the reference coordinate system so that the display direction coincides with the specific display direction.

Accordingly, even in a case where imaging by MRI, PET, or the like is performed repetitively, when there is CT data of the same patient acquired in the past, all the imaging data of this patient can be aligned and displayed in the direction set on the CT data using the landmarks common with the CT data or the markers attached to the common positions with the CT data. The imaging data can be therefore utilized efficiently.
Also, the original imaging data itself has a large amount of data. Hence, retrieving sets of data acquired by different imaging devices every single time and overlaying these sets of data require time and effort for storage management and operation processing of the data. However, the image data generated in the past is normal two-dimensional image data having a small amount of data, and it is normally attached to electronic medical charts and can be retrieved easily. In addition, by storing the data of each reference plane coordinate system and variable data alone, for example, a PET image in the direction of a CT image attached to a medical chart can be readily formed, and the two images can be attached side by side for comparative evaluation. It is therefore possible to save efforts and hours of work for the management of original data and for the work to overlay two sets of data each having a large mount of data and the system costs. The image data can be therefore utilized for clinical care more effectively and efficiently.

Further, for example, once a transformation coefficient is found from CT data and PET data acquired with markers attached to the common positions, as long as another set of PET data acquired earlier or later at a different time without attaching any markers has features common with the firstly-mentioned PET data attached with the markers on the extracted images, the secondly-mentioned set of PET data can be displayed in the display direction according to the CT data by correlating these two sets of the PET data with each other on the basis of the features. In short, is it possible to display another set of PET data in the same direction so that it can be utilized efficiently as materials for examination and evaluation.

### Brief Description of the Drawings

FIG. 1 is a block diagram showing the configuration of a computer system that executes a medical image display program according to one embodiment of the invention.
FIG. 2 is a flowchart detailing a display operation by the medical image display program according to one embodiment of the invention executed by the computer system.
FIG. 3 is another flowchart detailing the display operation by the medical image display program according to one embodiment of the invention executed by the computer system.
FIG. 4 is a schematic view showing an example of the bone image on a CT image.
FIG. 5 is a schematic view showing the bone image in a display direction CT1.
FIG. 6 is a schematic view showing an example of the skin image on a CT image.
FIG. 7 is a schematic view showing an image in a display direction CT2 according to a coordinate system 2.
FIG. 8 is a schematic view of a corrected image obtained by correcting the direction of the image in the display direction CT2 according to the coordinate system 2 shown in FIG. 7 so as to coincide with the direction shown in FIG. 5.
FIG. 9 is a schematic view showing an example of an MRI image.
FIG. 10 is a schematic view showing an example of an image in a display direction MRI2 according to a coordinate system 2'.
FIG. 11 is a schematic view of an image showing a state after the image in the display direction MRI2 according to the coordinate system 2' is corrected to an image in a display direction MRI1 according to a coordinate system 1'.

### Description of Reference Numerals and Signs

1: control portion
2: memory
3: reader and writer
4: operation portion
6: display device
7: CT data
8: MRI data
10: computer system

### Best Mode for Carrying Out the Invention

Hereinafter, one embodiment of the invention will be described with reference to the drawings.

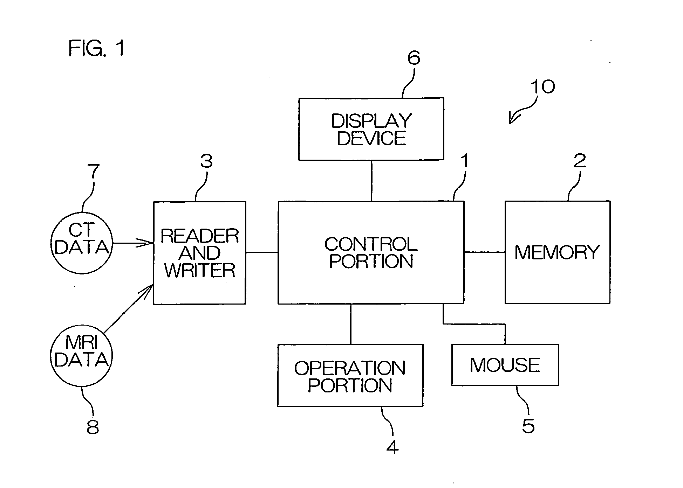

FIG. 1 is a block diagram showing the configuration of a computer system 10 that executes a medical image display program according to one embodiment of the invention. The computer system 10 is a known personal computer system or an office computer system.
The system 10 is provided with a control portion 1 including a CPU. A memory 2 (it can be of any type and examples include but not limited to a hard disk memory and a solid-state memory), a reader and writer 3, an operation portion (examples include but not limited to a keyboard and an operation panel) 4, a mouse 5 as an operation member, a display device (examples include but not limited to a liquid crystal display, a CRT display, and a plasma display) 6 are connected to the control portion 1.

When disk-shaped recoding media in which are recorded CT data 7 and MRI data 8 are loaded in the reader and writer 3, it reads out CT data and MRI data, respectively, from the disks and provides the read data to the control portion 1.
By using the reader and writer 3, it becomes possible to install the medical image display program according to one embodiment of the invention in this computer system. The computer system in which is installed the medical image display program becomes capable of displaying medical images obtained from the CT data 7 and the MRI data 8 as will be described below.

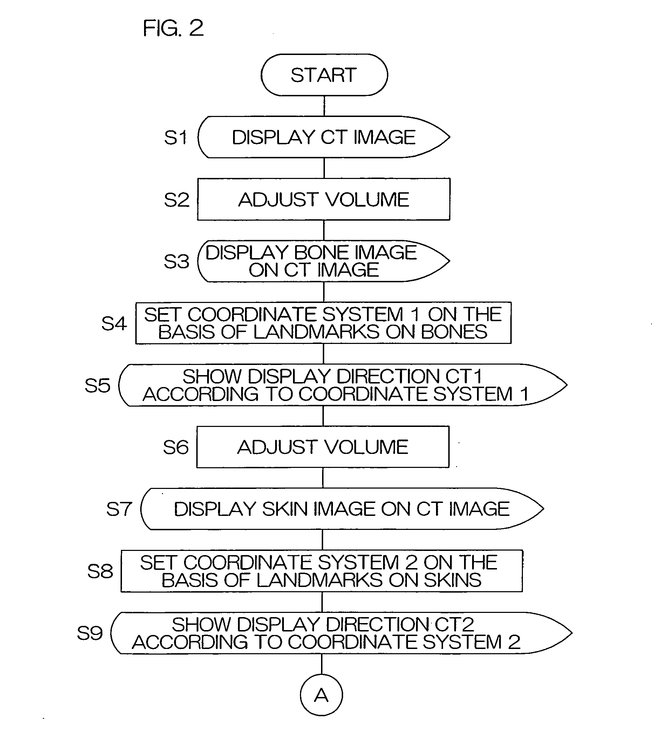

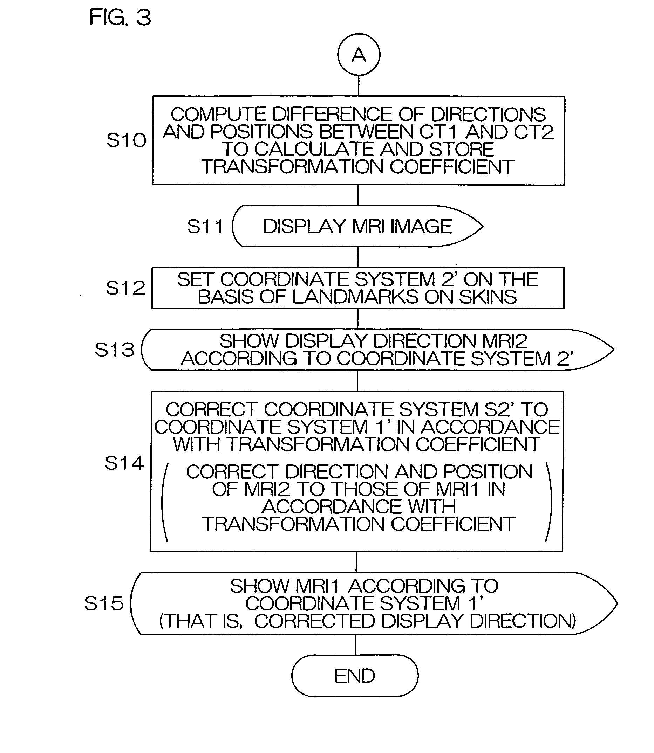

FIG. 2 and FIG. 3 show the flowcharts detailing a display operation by the medical image display program according to one embodiment of the invention executed by the computer system 10 shown in FIG. 1.
When the control starts, a CT image according to the CT data is displayed first on the display device 6 (Step S1).

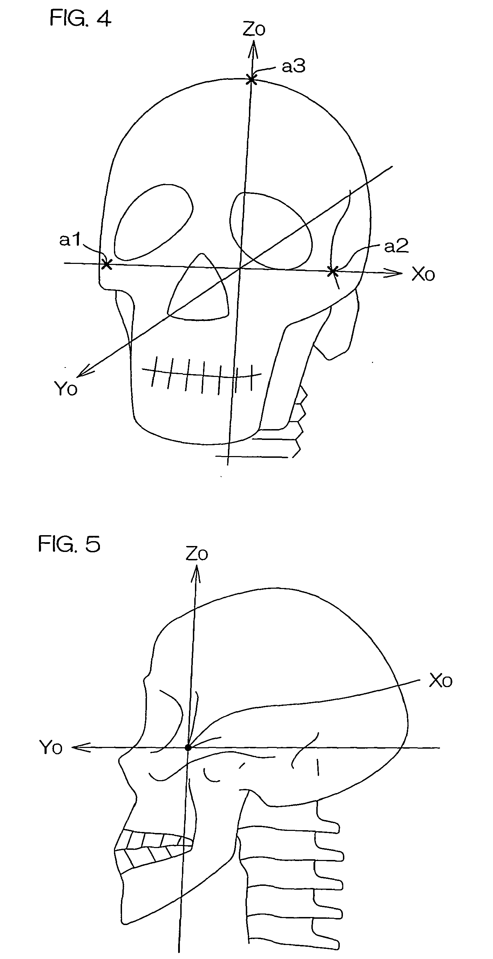

The user adjusts a display volume of the CT image using the operation portion 4 or the mouse 5 (Step S2), so that the bone image is shown on the CT image being displayed on the display device 6 (Step S3).
An example of the bone image on the CT image displayed in Step S3 is shown in FIG. 4.
In the bone image on the CT image in FIG. 4, by specifying a plurality of landmarks (anatomical features) present on the bones, a first reference coordinate system (coordinate system 1) can be set in the CT image of FIG. 4 (Step S4). For example, by specifying the highest points a1 and a2 of the superior margins of the external auditory meatuses on both the right and left lateral sides of the human skull, an axis passing through the both specified points can be defined as a reference axis X₀. Further, by specifying the landmark a3 on the coronal suture in the parietal region in the anatomical chart, an axis passing through the specified point a3 and orthogonal to the reference axis X₀ can be defined as another reference axis Z₀. Furthermore, a line passing through the intersection of X₀ and Z₀ and orthogonal to the both reference axes can be defined as still another reference axis Y₀.

The method of setting the first reference coordinate system on the basis of the landmarks on the bones is not limited to the method described above, and another method is also available. For example, it may be a method as is disclosed in Patent Document 1 specified above by which a first reference coordinate system is set by setting a reference plane on the basis of the landmarks.
Subsequently, the CT image is displayed on the display device 6 in a desired direction, for example, in a direction for the left lateral view to appear, according to the first reference coordinate system (coordinate system 1)(Step S5). Let this display direction be CT1. FIG. 5 shows the bone image in the display direction CT1.

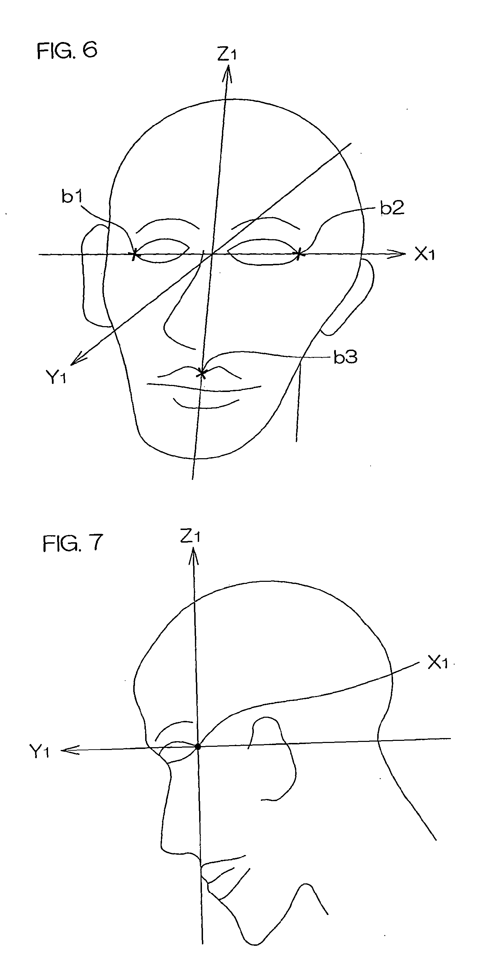

Subsequently, the direction of the CT image displayed on the display device 6 is set to an arbitrary direction and the display volume is adjusted (Step S6), so that the skin image is shown on the CT image (Step S7). FIG. 6 shows an example of the skin image on the CT image.
The user confirms the skin image and specifies a plurality of landmarks on the skins. A second reference coordinate system (coordinate system 2) can be thus set (Step S8).

For example, by specifying the lateral orbits b1 and b2 of both the right and left eyes in FIG. 6, a straight line passing through b1 and b2 can be defined as a reference axis X₁.
Further, by specifying the top center b3 of the lips, an axis passing through b3 and orthogonal to the reference axis X₁ can be defined as another reference axis Z₁.
Furthermore, an axis passing through the intersection of the reference axes X₁ and Z₁ and orthogonal to the both reference axes X₁ and Z₁ can be defined as still another reference axis Y₁.

The method of setting these reference axes is not limited to the content described above, either. The axes may be set by another method using a plurality of landmarks on the skins.
Subsequently, by displaying the left lateral view of the human head, that is, an image in a display direction CT2, is displayed according to the second reference coordinate system (coordinate 2) (Step S9), it is possible to obtain the CT image shown in FIG. 7.
As is obvious from FIG. 7, the second reference axis (reference axis 2) is the reference axis on the basis of the landmarks on the skins, and the display direction of the image of the human head differs significantly from the display direction of the bone image shown in FIG. 5.

Accordingly, the image (shown in FIG. 5) in the display direction CT1 according to the first reference coordinate system (coordinate 1) and the image (shown in FIG. 7) in the display direction CT2 according to the second reference coordinate system (coordinate system 2) differ significantly in display direction. In a case where the user wishes to make an evaluation by comparing the both images with each other, because the coordinate system 1 and the coordinate system 2 are different, the display directions between the both images are also different, and such a difference poses a problem that it is difficult to compare one image with the other.

To overcome this problem, the program of this embodiment is configured to compute differences of the directions and the positions between the image in the display direction CT1 and the image in the display direction CT2 to calculate and store a transformation coefficient in Step S10.
To be more concrete, a difference between the reference coordinate system X₀, Y₀, and Z₀ (coordinate system 1) of the image in the display direction CT1 and X₁, Y₁, and Z₁, which form the reference coordinate system (second reference coordinate system, coordinate system 2) in the second display direction CT2, is computed to find angular differences Δθx, Δθy, and Δθz and positional differences Δtx, Δty, and Δtz, which are used to calculate a transformation coefficient Tc. The transformation coefficient Tc is then stored.

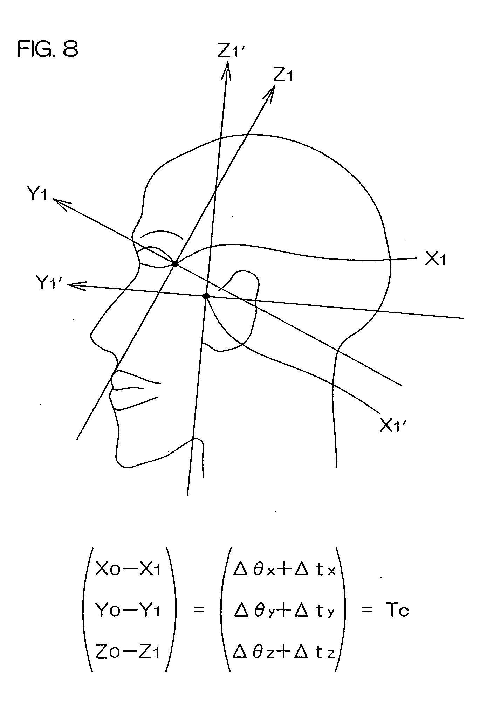

The image displayed according to the reference coordinate system (second reference coordinate system, coordinate system 2) in the second display direction CT2 is corrected using the transformation coefficient Tc so as to coincide with the image in the display direction according to the coordinate system 1. An image shown in FIG. 8 is thus obtained. As is obvious from the comparison between the image in FIG. 8 and the image in FIG. 5, the both images are in almost the same display direction. It thus becomes possible to compare one image with the other easily and correctly.

Subsequently, a different set of data from the CT data, for example, MRI data 8, is read out by the reader and writer 3, and an MRI image according to the read MRI data is displayed (Step S11).
Because no bone is shown on the MRI image, a third reference coordinate system (coordinate system 2') is set by specifying a plurality of landmarks on the skins on the basis of the skin image (Step S12).

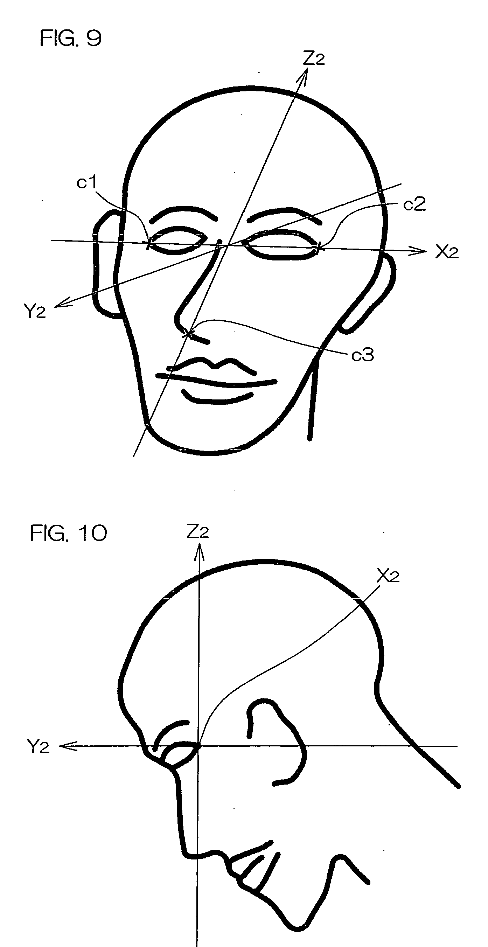

An example of the MRI image is shown, for example, in FIG. 9. In the MRI image shown in FIG. 9, the lateral orbits c1 and c2 of both the right and left eyes are specified and a straight line passing through these c1 and c2 is defined as a reference axis X₂. Further, by specifying the tip c3 of the nose, a straight line passing through the tip c3 and orthogonal to the reference axis X₂ is defined as another reference axis Z₂. Furthermore, a straight line passing through the intersection of the reference axes X₂ and Z₂ and orthogonal to the both reference axes is defined as still another reference axis Y₂.

It should be appreciated that the method of determining the reference axes X₂, Y₂, and Z₂ in this case is not limited to the method described above. As with the method of determining the reference axes above, it can be any method as long as it is a method of setting the reference axes on the basis of a plurality of landmarks on the skins.
As has been described, the third reference coordinate system (coordinate system 2') on the basis of the landmarks on the skins on the MRI image is set (Step S12).

Subsequently, the direction of the image of the human head is changed according to the third reference coordinate system (coordinate system 2') so that the left lateral view of the head is shown. The image in a display direction MRI2 shown in FIG. 10 is thus obtained.
This image is an image inclined frontward. In a case where this image is to be compared, for example, with the CT image shown in FIG. 5, it is extremely difficult to compare one image with the other.
To overcome this problem, the program of this embodiment is configured to correct the third reference coordinate system (coordinate system 2') in accordance with the transformation coefficient Tc calculated in Step S10 (Step S14).

More specifically, the direction and the position of the third reference coordinate system (coordinate system 2') are corrected by multiplying the third reference coordinate system (coordinate system 2') by the transformation coefficient Tc. The third reference coordinate system (coordinate system 2') is thus corrected to a fourth reference coordinate system (coordinate system 1').

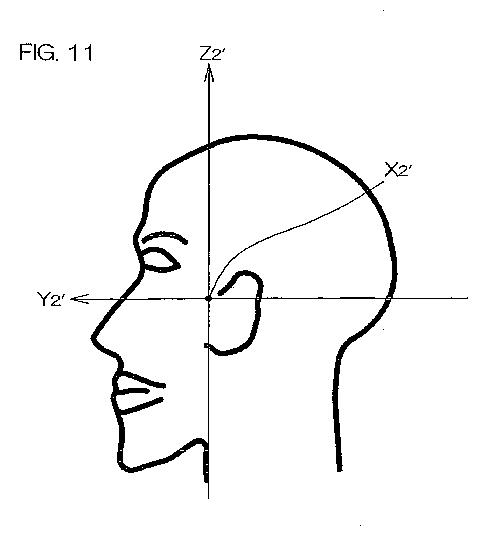

FIG. 11 shows the reference axes of the fourth reference coordinate system (coordinate system 1') set by transformation and an image in the direction of MRI1 displayed according to the coordinate system 1'. The display direction of the MRI image displayed according to the fourth reference coordinate system (coordinate system 1') set by transformation and indicated by reference axes X₃, Y₃, and Z₃ is a display direction MRI1. This image is therefore an image effective (suitable) to be compared with the CT image shown in FIG. 5 in direction and position.

The embodiment above has described the method of constructing a common coordinate system on the basis of the transformation coefficient between a CT image and an MRI image. It should be appreciated, however, that the invention can be used when common coordinates are constructed between images obtained by different imaging devices or imaging methods, such as between a CT image and a PET image or SPECT image.
In addition, the embodiment above is also used effectively in a case where a coordinate system on the basis of contrast markers that are used when common landmarks cannot be found on images obtained by different imaging devices or imaging methods is transformed to a coordinate system on the basis of an anatomical direction determined on data having high anatomical extraction, such as CT data and MRI data.

In addition, it should be appreciated that the invention can be modified in various manners within the scope of the appended claims.

## Claims

1. A display method of a medical image according to image data acquired by different imaging methods, comprising the steps of:
displaying a first image according to first medical three-dimensional image data of a patient acquired by a first imaging method;
setting a first reference coordinate system used to determine a direction of the first image on the basis of a plurality of first landmarks included in the first image;
setting a second reference coordinate system used to determine the direction of the first image on the basis of a plurality of second landmarks included in the first image and having at least one landmark different from the first landmarks;
computing a difference of directions between the first reference coordinate system and the second reference coordinate system and calculating a transformation coefficient between the both coordinate systems on the basis of the difference;
displaying a second image according to second medical three-dimensional image data of the patient acquired by a second imaging method;
setting a third reference coordinate system used to determine a direction of the second image on the basis of a plurality of third landmarks included in the second image;
correcting the third reference coordinate system to a fourth reference coordinate system using the transformation coefficient that has been calculated; and
displaying the second image in a direction according to the fourth reference coordinate system set by correction.

2. A display method of a medial image, comprising the steps of:
preparing a transformation coefficient on the basis of a difference between a pre-calculated coordinate system in reference to landmarks on hard tissues represented by bones and a pre-calculated coordinate system in reference to landmarks on soft tissues represented by skins;
constructing a first reference coordinate system used to determine a direction of an image on the basis of a plurality of landmarks on an image showing the soft tissues in a case where a medical image of a patient taken by a predetermined imaging method is an image that shows the soft tissues represented by the skins but does not show the hard tissues represented by the bones;
correcting the first reference coordinate system to a second reference coordinate system using the transformation coefficient; and
displaying an image of the patient in a direction according to the second reference coordinate system.

3. A display method of a medical image according to image data acquired by different imaging methods, comprising the steps of:
computing a difference between two coordinate systems set on first medical three-dimensional image data acquired by a first imaging method and calculating a transformation coefficient between the both coordinate systems on the basis of the difference;
setting a coordinate system common with either one of the two coordinate systems on second medical three-dimensional image data acquired by a second imaging method; and
correcting the coordinate system set on the second medical three-dimensional image data using the transformation coefficient that has been calculated.

4. The display method of a medical image according to any one of claims 1 through 3, wherein the step of setting or constructing the coordinate system using a display image of anatomical landmarks or imaging markers displayed on the image.

5. A medical image display program that causes a computer to display a medical image, comprising the steps of:
displaying a first image according to first medical three-dimensional image data of a patient acquired by a first imaging method;
setting a first reference coordinate system used to determine a direction of the first image on the basis of a plurality of first landmarks included in the first image;
setting a second reference coordinate system used to determine the direction of the first image on the basis of a plurality of second landmarks included in the first image and having at least one landmark different from the first landmarks;
computing a difference of directions between the first reference coordinate system and the second reference coordinate system and calculating a transformation coefficient between the both coordinate systems on the basis of the difference;
displaying a second image according to second medical three-dimensional image data of the patient acquired by a second imaging method;
setting a third reference coordinate system used to determine a direction of the second image on the basis of a plurality of third landmarks included in the second image;
correcting the third reference coordinate system to a fourth reference coordinate system using the transformation coefficient that has been calculated; and
displaying the second image in a direction according to the fourth reference coordinate system set by correction.

6. A medical image display program that causes a computer to display a medical image, comprising the steps of:
preparing a transformation coefficient on the basis of a difference between a pre-calculated coordinate system in reference to landmarks on hard tissues represented by bones and a pre-calculated coordinate system in reference to landmarks on soft tissues represented by skins;
constructing a first reference coordinate system used to determine a direction of an image on the basis of a plurality of landmarks on an image showing the soft tissues in a case where a medical image of a patient taken by a predetermined imaging method is an image that shows the soft tissues represented by the skins but does not show the hard tissues represented by the bones;
correcting the first reference coordinate system to a second reference coordinate system using the transformation coefficient; and
displaying an image of the patient in a direction according to the second reference coordinate system.

7. A medical image display program that causes a computer to display a medical image, comprising the steps of:
computing a difference between two coordinate systems set on first medical three-dimensional image data acquired by a first imaging method and calculating a transformation coefficient between the both coordinate systems on the basis of the difference;
setting a coordinate system common with either one of the two coordinate systems on second medical three-dimensional image data acquired by a second imaging method; and
correcting the coordinate system set on the second medical three-dimensional image data using the transformation coefficient that has been calculated.

8. The medical image display program that causes a computer to display a medical image according to any one of claims 5 through 7, wherein the step of setting or constructing the coordinate system using a display image of anatomical landmarks or imaging markers displayed on the image.
